(19) Europäisches Patentamt
European Patent Office
Office européen des brevets

(11) Publication number: 0 174 085 A2

# (12) EUROPEAN PATENT APPLICATION

(21) Application number: 85305285.0

(22) Date of filing: 25.07.85

(51) Int. Cl.[4]: C 07 C 103/82
C 07 C 103/30, C 07 D 211/58
C 07 D 211/26, C 07 D 213/82
C 07 C 121/50, C 07 D 317/60
//C07C121/43, C07C87/20,
C07C103/48, C07C91/08,
C07C87/22, C07D211/74,
C07D211/22, C07D211/18

(30) Priority: 26.07.84 GB 8419103

(43) Date of publication of application: 12.03.86 Bulletin 86/11

(84) Designated Contracting States: AT BE CH DE FR GB IT LI LU NL SE

(71) Applicant: BEECHAM - WUELFING GmbH & Co. KG
Stresemannallee 6 P.O. Box 25
D-4040 Neuss(DE)

(72) Inventor: Jozic, Ljerka
An der Bismarck-Schule 4B
D-3000 Hanover 1(DE)

(74) Representative: Valentine, Jill Barbara et al,
Beecham Pharmaceuticals Patents & Trade Marks Dept.
Great Burgh Yew Tree Bottom Road
Epsom Surrey KT18 5XQ(GB)

## (54) Bisarylamides of N,N'-di-]1-aminoalkyl-2[-alkylene diamines having a cardiovascular activity.

(57) Disclosed are compounds of formula (I) and pharmaceutically acceptable salts thereof:

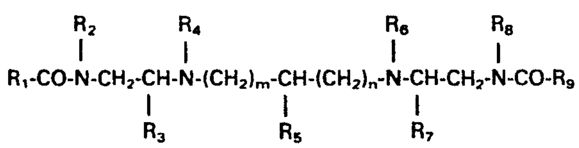

(I)

wherein:

$R_1$ and $R_9$ are independently phenyl, phenyl $C_{1-4}$ alkyl, phenyl $C_{2-4}$ alkenyl or pyridyl, the aromatic moiety optionally substituted by one, two or three of halogen, trifluoromethyl, $C_{1-4}$ alkoxy, $C_{1-4}$ alkyl, cyano, hydroxy, nitro, $NR_{10}R_{11}$ or $O_2SNR_{10}R_{11}$ wherein $R_{10}$ and $R_{11}$ are independently hydrogen or $C_{1-6}$ alkyl or together are $C_{3-6}$ polymethylene, or disubstituted at adjacent carbon atoms by $C_{1-2}$ alkylenedioxy; $C_{3-8}$ cycloalkyl; or aliphatic heterocyclyl having up to six ring atoms the heteroatom(s) being selected from oxygen, sulphur or nitrogen;

m and n are independently integers of 0 to 3, such that m + n is 0 to 3; and $R_2$ and $R_8$ are independently hydrogen $C_{1-4}$ alkyl or phenyl $C_{1-4}$ alkyl, $R_3$ is $C_{1-4}$ alkyl optionally substituted by hydroxy or thiol or phenyl $C_{1-4}$ alkyl, or together with $R_4$ is $C_{3-6}$ polymethylene, $R_4$ is hydrogen, $C_{1-4}$ alkyl, phenyl $C_{1-4}$ alkyl or as defined with $R_3$, or together with $R_5$ is $-(CH_2)_p-$where p is an integer from 2 to 6 such that m + p is 3 to 6, $R_5$ is hydrogen, $C_{1-4}$ alkyl or as defined with $R_4$, or together with $R_6$ is $-(CH_2)_q-$where q is an integer from 2 to 6 such that n + q is 3 to 6, $R_6$ is hydrogen, $C_{1-4}$ alkyl, phenyl $C_{1-4}$ alkyl or as defined with $R_5$, or together with $R_7$ is $C_{3-6}$ polymethylene, and $R_7$ is $C_{1-4}$ alkyl optionally substituted by hydroxy or thiol, phenyl $C_{1-4}$ alkyl or as defined with $R_6$, in which any phenyl moiety in $R_2$,$R_3$,$R_4$,$R_6$,$R_7$ and $R_8$ is optionally substituted by one or two of halogen, trifluoromethyl, $C_{1-4}$ alkoxy or $C_{1-4}$ alkyl.

Such compounds have been found to possess cardiovascular activity, in particular calcium antagonistic activity which indicates that they are of potential use in the treatment of angina.

EP 0 174 085 A2

B1684

# NOVEL COMPOUNDS

The present invention relates to novel compounds having pharmacological activity, to a process for their preparation, to pharmacological compositions containing them and to their use in the treatment of mammals.

United States Patent No. 4021473 describes esters of alkoxy substituted benzoic acid which have antiarrhythmic activity.

A structurally distinct class of compounds has now been discovered which compounds are substituted amides of NN'-di-[1-aminoalkyl-2]-ethylene diamine. Such compounds have been found to possess cardiovascular activity, in particular calcium antagonistic activity which indicates that they are of potential use in the treatment of angina.

Accordingly the present invention provides a compound of formula (I) or a pharmaceutically acceptable salt thereof:

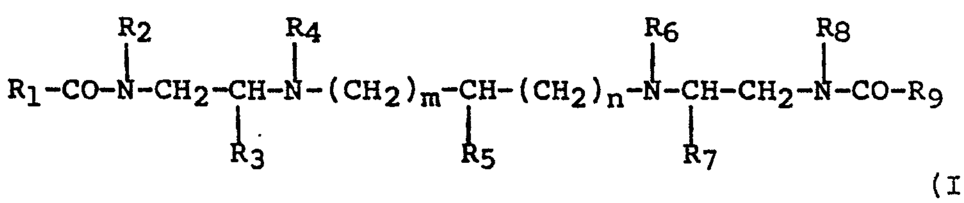

(I)

wherein:

$R_1$ and $R_9$ are independently phenyl, phenyl $C_{1-4}$ alkyl, phenyl $C_{2-4}$ alkenyl or pyridyl, the aromatic moiety optionally substituted by one, two or three of halogen, trifluoromethyl, $C_{1-4}$ alkoxy, $C_{1-4}$ alkyl, cyano, hydroxy, nitro, $NR_{10}R_{11}$ or $O_2SNR_{10}R_{11}$ wherein $R_{10}$and $R_{11}$ are independently hydrogen or $C_{1-6}$ alkyl or together are $C_{3-6}$ polymethylene, or disubstituted at adjacent carbon atoms by $C_{1-2}$ alkylenedioxy; $C_{3-8}$ cycloalkyl; or aliphatic heterocyclyl having up to six ring atoms the heteroatom(s) being selected from oxygen, sulphur or nitrogen;

m and n are independently integers of 0 to 3, such that m + n is 0 to 3; and

$R_2$ and $R_8$ are independently hydrogen $C_{1-4}$ alkyl or phenyl $C_{1-4}$ alkyl, $R_3$ is $C_{1-4}$ alkyl optionally substituted by hydroxy or thiol or phenyl $C_{1-4}$ alkyl, or together with $R_4$ is $C_{3-6}$ polymethylene, $R_4$ is hydrogen, $C_{1-4}$ alkyl, phenyl $C_{1-4}$ alkyl or as defined with $R_3$, or together with $R_5$ is $-(CH_2)_p-$ where p is an integer from 2 to 6 such that m + p is 3 to 6, $R_5$ is hydrogen, $C_{1-4}$ alkyl or as defined with $R_4$, or together with $R_6$ is $-(CH_2)_q-$ where q is an integer from 2 to 6 such that n + q is 3 to 6, $R_6$ is hydrogen, $C_{1-4}$ alkyl, phenyl $C_{1-4}$ alkyl or as defined with $R_5$, or together with $R_7$ is $C_{3-6}$ polymethylene, and $R_7$ is $C_{1-4}$ alkyl optionally substituted by hydroxy or thiol, phenyl $C_{1-4}$ alkyl or as defined with $R_6$, in which any phenyl moiety in $R_2$,$R_3$,$R_4$,$R_6$,$R_7$ and $R_8$ is optionally substituted by one or two of halogen, trifluoromethyl, $C_{1-4}$ alkoxy or $C_{1-4}$ alkyl.

Suitable values for $R_1$ and $R_9$ include phenyl,benzyl, styryl, pyridyl, cyclopentyl, cyclohexyl, cycloheptyl and phenyl, benzyl or styryl di-substituted by methylenedioxy or substituted by one, two or three of fluoro, chloro, bromo, trifluoromethyl, methoxy, ethoxy, n- or iso-propoxy, methyl, ethyl, n - or iso-propyl, cyano, hydroxy or amino optionally substituted by one or two methyl groups. Preferably $R_1$ and $R_9$ are phenyl or substituted phenyl. $R_1$ and $R_9$ are preferably the same group.

Suitable values for $R_2$, $R_4$, $R_6$ and $R_8$ include hydrogen, methyl, ethyl, n - and iso-propyl or benzyl. Preferably $R_2$ and $R_8$ are the same and are hydrogen or $C_{1-4}$ alkyl. Most preferably, $R_2$ and $R_8$ are hydrogen. Preferably $R_4$ and $R_6$ are the same and are hydrogen or $C_{1-4}$ alkyl. Most preferably, $R_4$ and $R_6$ are both methyl.

Suitable values for $R_5$ include hydrogen, methyl, ethyl, n- and iso-propyl, and n, iso -, sec - and t-butyl. Preferably $R_5$ is hydrogen.

Suitable values for $R_3$ and $R_7$ include methyl, ethyl, n- and iso-propyl, n-, iso-, sec- and t-butyl, hydroxymethyl, hydroxyethyl, thiomethyl and thiolethyl. Preferably $R_3$ and $R_7$ are the same and are $C_{1-4}$ alkyl, most preferably ethyl, n-propyl or iso-butyl.

Suitable values for $R_3/R_4$ and $R_6/R_7$ when $C_{3-6}$ polymethylene include $-(CH_2)_3-$ and $-(CH_2)_4-$, preferably $-(CH_2)_4-$.

Suitable values for m + p and n + q include 3 and 4, preferably 4.

Suitable values for p and q include 2,3 and 4, preferably 2.

Suitable values for m + n include 0, 1 or 2. Preferably m + n is 1 or 2.

There is a favourable group of compounds within formula (I) of formula (II):

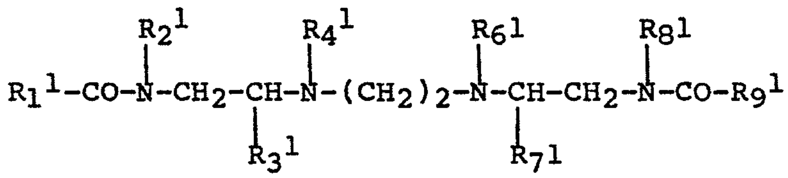

(II)

wherein:

$R_1^1$ and $R_9^1$ are pyridyl, phenyl, benzyl or styryl, optionally substituted in the phenyl ring as defined in formula (I);

$R_2^1$, $R_4^1$, $R_6^1$ and $R_8^1$ are hydrogen or $C_{1-4}$ alkyl; and $R_3^1$ and $R_7^1$ are $C_{1-4}$ alkyl.

Suitable and preferred values for $R_1^1$ to $R_4^1$ and $R_6^1$ to $R_9^1$ are as described for the corresponding variables under formula (I).

Prefered values for $R_1^1$ and $R_9^1$ include 3,5-dichlorophenyl, 3-chlorophenyl, 3,4-dimethylphenyl, 3,4-methylenedioxyphenyl, 3,4-dimethoxyphenyl and 3,4,5-trimethoxyphenyl.

There is another group of compounds within formula (I) of formula (IIa):

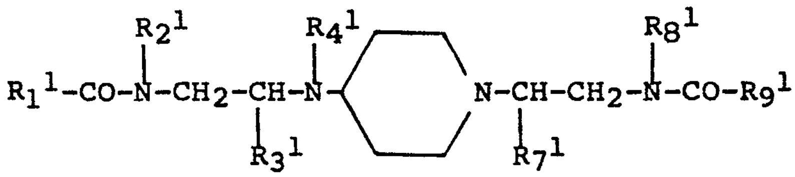

(IIa)

wherein $R_1^1$ to $R_4^1$ and $R_7^1$ to $R_9^1$ are as defined in formula (II).
Suitable and preferred values for $R_1^1$ to $R_4^1$ and $R_7^1$ to $R_9^1$ are as described for the corresponding variables under formula (II).

It will of course be realised that the compounds of formula (I) possess two chiral centres and therefore exist in more than one stereoisomeric form. The invention extends to any of the stereoisometric forms, including enantiomers of the compounds of the invention and to mixtures thereof, including racemates. The different stereoisomeric forms may be separated or resolved one from the other by the usual methods or any given isomer may be obtained by stereospecific or asymmetric syntheses.

The pharmaceutically acceptable salts of the compounds of formula (I) include acid addition salts with conventional acids such as hydrochloric, hydrobromic, boric, phosphoric, sulphuric and pharmaceutically acceptable organic acids such as acetic, tartaric, maleic, citric, succinic, benzoic, ascorbic, methanesulphonic, α-keto-glutaric, α-glycerophosphoric, and glucose-1-phosphoric acids. Preferably the acid addition salt is a hydrochloride.

Pharmaceutically acceptable salts also include quaternary salts. Examples of quaternary salts include such compounds quaternised by compounds such as $R_{12}$-T wherein $R_{12}$ is $C_{1-6}$ alkyl, phenyl-$C_{1-6}$ alkyl or $C_{5-7}$ cycloalkyl, and T is a radical corresponding to an anion of an acid. Suitable examples of $R_{12}$ include methyl, ethyl and _n_- and _iso_- propyl; and benzyl and phenethyl. Suitable T include halide such as chloride, bromide and iodide.

Pharmaceutically acceptable salts also include pharmaceutically acceptable N-oxides, and the invention extends to these.

The compounds of the formula (I) and their pharmaceutically acceptable salts may also form solvates with pharmaceutically acceptable solvates and the invention extends to these.

It will also be realised that salts of the compounds of the formula (I) which are not pharmaceutically acceptable may be useful as intermediates in the preparation of pharmaceutically acceptable salts of

compounds of the formula (I) or the compounds of the formula (I) themselves, and as such form an aspect of the present invention.

The invention also provides a process for the preparation of a compound of formula (I) or a pharmaceutically acceptable salt thereof, which process comprises reacting a compound of formula (III):

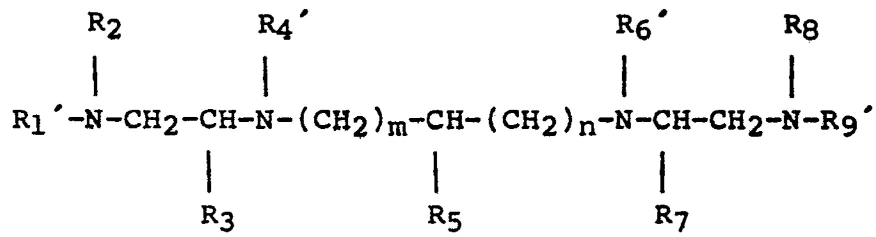

(III)

with a compound of formula (IV):

$$L\text{-}CO\text{-}R_{13} \qquad (IV)$$

wherein $R_4'$ and $R_6'$ are $R_4$ and $R_6$ or N-protecting groups, one of $R_1'$ and $R_9'$ is hydrogen and the other is $-COR_1$ or $-COR_9$ respectively or when $R_1$ and $R_9$ are the same group, $R_1'$ and $R_9'$ may both be hydrogen; $R_{13}$ is $R_9$ when $R_1$ is hydrogen and/or $R_1$ when $R_9$ is hydrogen and L is a leaving group, and thereafter removing $R_4'$ and/or $R_6'$ when protecting groups and/or optionally converting $R_2$, $R_4$, $R_6$ or $R_8$ when hydrogen to other $R_2$, $R_4$, $R_6$, or $R_8$ and/or optionally forming a pharmaceutically acceptable salt thereof.

The leaving group L is a group readily displaceable by a nucleophile. Suitable examples of L are hydroxy, halogen such as chloro and bromo and acyloxy such as $C_{1-4}$ alkanoyloxy, $C_{1-4}$ alkoxycarbonyloxy and activated hydrocarbyloxy such as pentachlorophenoxy.

If the leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as -10 to 100°C, for example 0 to 80°C.

If the leaving group is a halide, then the reaction is preferably carried out at a non-extreme temperature in an inert non-hydroxylic solvent, such as benzene, toluene or diethyl ether. It is also preferably carried out in the presence of an acid acceptor, such as an organic base, in particular a tertiary amine, such as triethylamine, trimethylamine, pyridine or picoline, some of which can also function as a solvent. Alternatively, the acid acceptor can be inorganic, such as calcium carbonate, sodium carbonate or potassium carbonate.

If the leaving group is acyloxy, then the reaction is preferably carried in substantially the same manner as if the leaving group were hydroxy. Suitable examples of acyloxy leaving groups include $C_{1-4}$ alkanoyloxy, mesyloxy, tosyloxy and triflate.

If the leaving group is $C_{1-4}$ alkoxycarbonyloxy, then the reaction is preferably carried out in an inert solvent, such as methylenechloride, at a non-extreme temperature in the presence of an acid acceptor, such as triethylamine.

If the leaving group is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

It will be appreciated that, when $R_1'$ and $R_9'$ are both hydrogen in the compound of formula (III) i.e. when $R_1$ and $R_9$ are the same group in the compound of formula (I), two molar equivalents of the compound of formula (IV) are necessary to complete the reaction to give the desired compound of formula (I).

Examples of N-protecting groups include $C_{1-6}$ alkanoyl, for example acetyl, propionyl, n- and iso-butyryl and 2,2-dimethylpropanoyl, benzoyl or benzene optionally substituted in the phenyl ring by one or two substituents selected from $C_{1-4}$ alkyl, $C_{1-4}$ alkoxy, trifluoromethyl, halogen or nitro; and $C_{1-4}$ alkoxycarbonyl, for example tert-butoxycarbonyl.

Conversion of protected amino to amino may be effected conventionally.

When the protecting group is $C_{1-6}$ alkanoyl or optionally substituted benzoyl as defined conversion to amino is conveniently effected by conventional base hydrolysis.

When the protecting group is $C_{1-4}$ alkoxycarbonyl or optionally substituted benzyl as defined, conversion to amino may be carried out conventionally, for example by hydrogenolysis. Suitable reactions are conventionally transition-metal catalysed hydrogenation, using for example palladium- or platinum-charcoal, at atmospheric pressure or a light excess thereover. A dry, inert, polar solvent such as dry ethanol and ambient temperatures are apt.

Subsequent conversion of $R_2$,$R_4$,$R_6$ or $R_8$ when hydrogen to other $R_2$,$R_4$,$R_6$ or $R_8$ may be carried out by conventional amine alkylation, for example with the appropriate aldehyde or ketone in a solvent such as acetonitrile, in the presence of a reducing agent such as an alkaline borohydride e.g. sodium cyanoborohydride.

Pharmaceutically acceptable salts including N-oxides of the compounds of formula (I) may be formed conventionally. The salts may be formed for example by reaction of the base compound of formula (I) with a pharmaceutically acceptable organic or inorganic acid.

N-oxides are produced by reaction of a compound of formula (I) with an organic peracid, such as m-chloroperbenzoic acid in, for example, a chlorinated hydrocarbon solvent at below ambient temperature.

Quaternary ammonium salts may be prepared by reaction of a compound of formula (I) with the appropriate alkyl, aryl or aralkyl, chloride, bromide or iodide. This reaction may be carried out in a solvent, such as acetone, methanol, ethanol or dimethylformamide, at ambient or elevated temperature with or without pressure.

Racemates of compounds of the formula (I) may be resolved conventionally, e.g., by salification with a chiral acid if appropriate and separation of the resultant salts.

Alternatively, either may be synthesised from corresponding chiral preceeding intermediates.

A compound of the formula (III) in which $R_1'$,$R_2$, $R_4'$, $R_5$, $R_6'$,$R_8$ and $R_9'$are each hydrogen, $R_3$ and $R_7$ are each ethyl, m is 1 and n is 0, has been described in Ann. N.Y. Acad. Sci. 135 (1966), p.686-710, R.G. Shepherd et al., on page 705.

The invention also provides intermediates of the formula (III) with the proviso that when $R_1'$, $R_2$, $R_4'$, $R_5$, $R_6'$, $R_8$ and $R_9'$ are each hydrogen, $R_3$ and $R_7$ are each ethyl and m is 1, n is other than 0.

Compounds of formula (III) wherein one of $R_1'$and $R_9'$is $-COR_1$ or $-COR_9$ respectively are prepared by reacting the compound of formula (V):

$$R_1''N-CH_2-\overset{R_2}{\overset{|}{C}}H-\underset{R_3}{\underset{|}{\overset{R_4}{\overset{|}{N}}}}-(CH_2)_m-\underset{R_5}{\underset{|}{C}}H-(CH_2)_n-\overset{R_6}{\overset{|}{N}}-\underset{R_7}{\underset{|}{C}}H-CH_2-\overset{R_8}{\overset{|}{N}}-R_9'' \qquad (V)$$

with a compound of formula (VI):

$$L_1\ COR_{14} \qquad\qquad (VI)$$

wherein one of $R_1''$and $R_9''$is hydrogen and the other is a protecting group, $R_{14}$ is $R_1$ or $R_9$ and $L_1$ is a leaving group; and therafter removing an $R_1''/R_9''$protecting group.

The leaving group $L_1$ is a group readily displaceable by a nucleophile. Suitable and preferred values for $L_1$ are described for L as hereinbefore defined. The reaction is carried out in an analogous manner to that between compounds of formulae (III) and (IV). De-protection may suitably be effected as described above for hydrogenolysable protected amino, under conventional conditions.

It will be appreciated that the necessity and order of deprotection/protection and the order of attachment at $R_1/R_9$ will depend in the nature of these groups and the appropriate reaction sequence selected.

Compounds of the formula (III) wherein $R_1'$and $R_9'$are both hydrogen, may be prepared by conventional procedures from compounds of formula (VII):

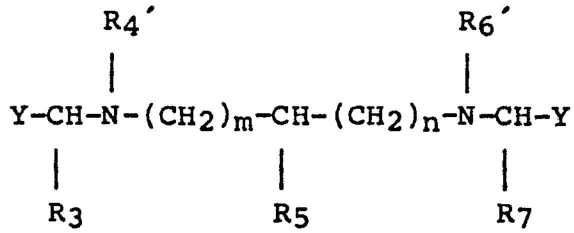 (VII)

where Y is a cyano group or a group $CH_2X$ where X is a leaving group such as halo or hydroxy. When Y is cyano, the procedure is a reduction with, for example, $LiAlH_4$ in diethylether, diborane, or hydrogen over Raney nickel in the presence of ammonia, when Y is $CH_2X$, the procedure is a nucleophilic substitution by ammonia or a primary or secondary amine under conventional conditions appropriate for the leaving group X. Thus, when X is hydroxy, it is first converted into a good leaving group such as mesylate tosylate or chloride.

Compounds of formulae (V) and (VII) are known from, or may be prepared by processes analogous to those described in, British Patent Specification 961317.

Compounds of formulae (IV) and (VI) are known compounds or may be prepared by processes analogous to those used for preparing known compounds.

The compounds of formula (I) have cardiovascular, in particular calcium antagonistic activity.

In order to utilise the activity of the compounds of formula (I) the invention also provides a pharmaceutical composition comprising a compound of formula (I), or a pharmaceutically acceptable salt thereof, or a solvate of any of the foregoing, and a pharmaceutically acceptable carrier.

Such compositions are prepared by admixture and are suitably adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art.

Suitable fillers for use include cellulose, mannitol, lactose and other similar agents. Suitable disintegrants include starch, polyvinylpyrrolidone and starch derivatives such as sodium starch glycollate.

Suitable lubricants include, for example, magnesium stearate. Suitable pharmaceutically acceptable wetting agents include sodium lauryl sulphate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

For parenteral administration, fluid unit dose forms are prepared containing a compound of the present invention and a sterile vehicle. The compound, depending on the vehicle and the concentration, can be

either suspended or dissolved. Parenteral solutions are normally prepared by dissolving the compound in a vehicle and filter sterilising before filling into a suitable vial or ampoule and sealing. Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound of the invention.

In addition such compositions may contain further active agents such as anti-hypertensive agents and diuretics.

As is common practice, the compositions will usually be accompanied by written or printed directions for use in the medical treatment concerned.

The invention further provides a method of treatment or prophylaxis of angina in mammals, such as humans, which comprises the administration of an effective amount of a compound of formula (I), or a pharmaceutically acceptable salt thereof, or a solvate of any of the foregoing as hereinbefore defined, to the sufferer.

An amount effective to treat the disorders hereinbefore described depends on the relative efficacies of the compounds of the formula (I), the nature and severity of the disorders being treated and the weight of the mammal. However, a unit dose will normally contain 1 to 100 mg for example 2 to 50 mg, of the compound of the invention. Unit doses will normally be administered once or more than once a day, for example 2,3,4,5 or 6 times a day, more usually 2 to 4 times a day, such that the total daily dose is normally in the range, for a 70 kg adult of 0.1 to 2500 mg, more usually 50 to 2000 mg, for example 10 to 75mg, that is in the range of approximately 0.002 to 35 mg/kg/day, more usually 1 to 30 mg/kg/day, for example 0.15 to 1 mg/kg/day.

The invention also provides a compound of formula (I), or a pharmaceutically acceptable salt thereof, or a solvate of any of the foregoing as hereinbefore defined, for use as an active therapeutic substance, and particularly for the treatment or prophylaxis of angina.

The following examples illustrate the invention and the following descriptions illustrate intermediates thereto.

Description 1

N, N'-dimethyl-N,N'-ethylene-bis-(2-amino-butane-nitrile) (D1)

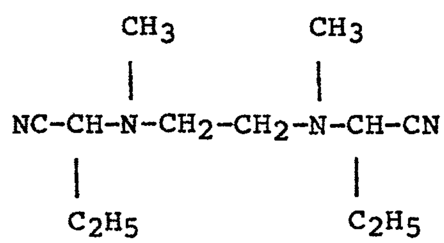

DI

123.8 g (1.4 mol) N,N'-dimethyl-ethylene-diamine and 137.7 g (2.81 mol) sodium cyanide were dissolved in a mixture of 250 ml ethanol, 300 ml water and 231.9 ml 37% HCl (d = 1.19) under stirring and cooling down to -10$^{o}$C, then 166.5 g (2.81 mol) freshly distilled propionaldehyde was added dropwise to the mixture and allowed to stand overnight at -20$^{o}$C. The reaction mixture was then neutralized with 297.9 g (2.81 mol) sodium carbonate and diluted with 400 ml water. The reaction mixture separated into two layers. The reaction product (upper layer) was decanted off and the remaining mixture was worked up in order to separate additional amounts of reaction product. The solid was filtered off and washed with 4 x 100 ml toluene. The mixture of filtrate and toluene extracts was stirred gently for 30 minutes, and the toluene layer containing reaction product was separated and combined with the reaction product decanted earlier. The toluene solution was dried over $Na_2SO_4$ and the solvent was removed *in vacuo*, yielding 229 g (73% of theory) D1.

Description 2

N,N'-dimethyl-N,N'-di-[2-(1-amino)-butyl]-ethylenediamine (D2)

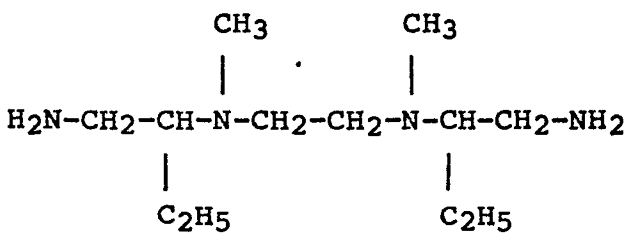

D2

132.4 g (1.19 mol) $LiAlH_4$ suspended in 1.5 l diethylether was heated under reflux for 1 hour. 132.4 g (0.59 mol) Nitrile D1 dissolved in 500 ml diethylether was added dropwise to the boiling $LiAlH_4$ solution. After the addition was complete the reaction mixture was stirred overnight at room temperature. 85 ml Water was dropped under cooling to the reaction mixture. The resulting solid was filtered off and washed with 300 ml diethylether and 300 ml methanol. The ether was removed by distillation, yielding 106.6 g compound D2. From a methanol solution was precipitated the chlorohydrate of compound D2 by the addition of saturated methanolic HCl. Yield 7.1 g hygroscopic tetrachlorohydrate of D2.

Description 3

Di-leucinethylester-oxamide (D3)

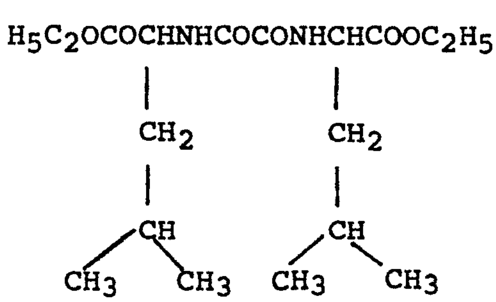

D3

A mixture of 181 g (1.14 mol) (±) leucine ethylester and 83.3 g (0.57 mol) diethyloxalate was heated for 3 hours under reflux. The reaction product was separated by suction and recrystallized from ethanol yielding 96g oxamide D3. An additional amount of product was precipitated from the reaction mixture-filtrate by dilution with water (1:1), yielding 60.6 g product which combined with the first precipitate gave 156.7 g oxamide D3. M.pt. 103°C (73.8 % of theory).

Description 4

N, N'-Di-(2-(1-oxy-4-methylpentyl)-ethylenediamine D4

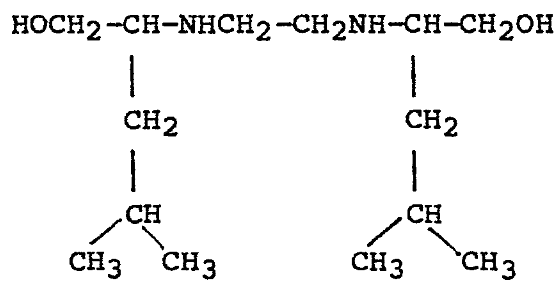

D4

65.8 g (1.68 mol) $LiAlH_4$ was suspended in 1 l diethyl ether and the suspension was heated for 1 hour under reflux. A solution of 156.7 g (0.42 mol) oxamide D3 in 1 l diethylether was added dropwise to the boiling suspension and then boiled for a further hour under reflux.

After reduction was complete the reaction mixture was cooled to room temperature and 111 ml water was added dropwise to the reaction mixture. The precipitate was sucked off and washed twice with 500 ml diethyl ether

and with 300 ml isopropanol. The filtrate was evaporated to dryness. The residue crystallized giving 98 g (87% of theory) D4.

Description 5

N,N'-dimethyl-N,N'-di-[2-(1-oxy-4-methyl)-pentyl]-ethylene diamine (D5)

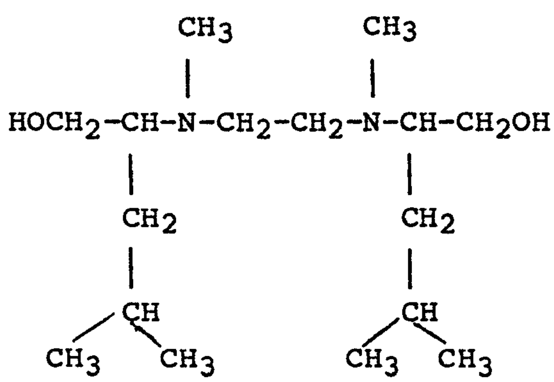

D5

To a mixture of 95 g (0.365 mol) D4,314 ml of 37% formaldehyde and 1000 ml acetonitrile was added 69.4 g (1.1 mol) $NaBH_3CN$ and, under cooling and stirring, 80 ml acetic acid. The reaction mixture was then stirred for 24 hours at room temperature. The solid was sucked off and the filtrate evaporated to dryness. The residue was dissolved in 400 ml chloroform and 200 ml water and made alkaline with NaOH (pH 12). The chloroform phase was separated, washed twice with 200 ml water and dried over $Na_2SO_4$. The solvent was removed *in vacuo* yielding 80.1 g oily residue D5 (77% of theory).

Description 6

N,N'-dimethyl-N,N'-di-[2-(1-chloro-4-methyl)-pentyl]-ethylene diamine (D6)

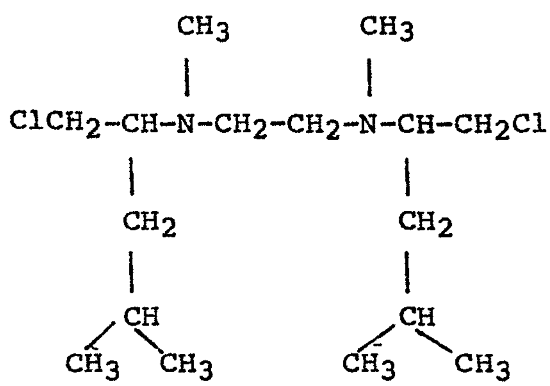

D6

To a cooled and stirred solution of 80.1 g (0.27 mol) D5 in 250 ml chloroform was added 55.5 g (0.466 mol) thionylchloride. The reaction mixture was heated for 4 hours under reflux. The solvent was evaporated *in vacuo*. The residue was hydrolysed with 18 ml water and dissolved in 500 ml isopropanol. The solution was boiled with charcoal and the product was precipitated by addition of 400 ml ethylacetate. The precipitate was sucked off and recrystallized from 200 ml isopropanol. Yield 67.7g D6 (63% of theory) as the dihydrochloride.

M.pt: 232°C (decomp.)

Empirical formula $C_{16}H_{34}N_2Cl_2.2HCl$ M.Wt.398.29

| Elem. anal.: | C | H | N | Cl |
|---|---|---|---|---|
| calc.: | 48.25 | 9.11 | 7.03 | 35.61 |
| found: | 48.33 | 9.11 | 7.03 | 35.58 |

Description 7

N,N'-dimethyl-N,N'-di-[2-(1-amino-4-methyl)-pentyl]-ethylene-diamine (D7)

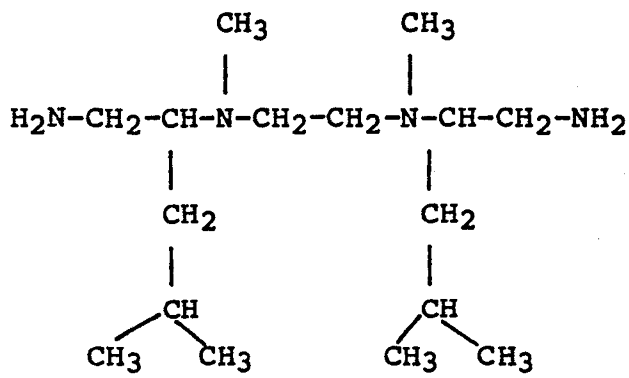

D7

A mixture containing 12g (0.03 mol) D6 as the dihydrochloride, 20.4 g (0.03 mol) 25% $NH_4OH$ (d = 0.91) and 200 ml methanol was stired for 4 hours at 30 - 50°C then 16 hours at room temperature. The solvent was removed *in vacuo* and the residue (pH 6.5 - 7.0) dissolved in 100 ml water was extracted with 100 ml chloroform to remove impurities. The aqueous solution was made alkaline to pH 12 and extracted with 2 x 100 ml chloroform. The organic extract was evaporated to dryness yielding 6.8 oily product D7 (84.4% of theory).

Description 8

1-[(1-Ethoxycarbonyl)propyl]-piperid-4-one (D8)

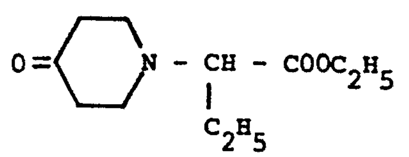

D8

To a solution of 60 g (0.6 mol) 4-oxo-piperidine and 60.7 g (0.6 mol) triethylamine in 200 ml abs. ethanol was dropped at room temperature a solution of 177 g

(0.6 mol) 2-bromobutyric acid ethyl ester in 200 ml abs. ethanol. The mixture was heated 5 hrs. under reflux. After cooling, the resulting solid was filtered off and the solvent was removed by distillation. The remaining mass was dissolved in 300 ml water and extracted with methylene chloride ( 3 x 200 ml). The organic phase was extracted with dilute HCl to separate the reaction product as the hydrochloride from unreacted starting material. The aqueous solution was made basic with saturated $Na_2CO_3$ solution, and the free base was extracted with methylene chloride (4 x 100 ml). The combined extracts were dried over $Na_2SO_4$, the solvent removed in vacuo yielding 66.1 g (52 % of theory) D8 as a red-brownish oil.

Description 9

1-[(1-Ethoxycarbonyl)propyl]-4-[(1-ethoxycarbonyl) propyl-amino]piperidine (D9)

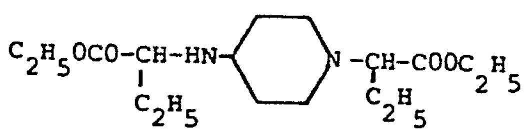

D9

To a solution of 69.1 g (0.45 mol) D, L-2-amino-butyric acid ethyl ester hydrochloride in 300 ml abs. methanol (over molecular sieve) was dropped a solution of 63.9 g (0.3 mol) D8 at room temperature. During the addition, 11.3 g (0.18 mol) $Na(CN)BH_3$ was added in 5 portions. The reaction mixture was stirred for 2 days until reaction was complete. The solid part was filtered off and the

filtrate evaporated. The residue was dissolved in methylene chloride, extracted with dilute NaOH (3 x 100 ml) and with water until the water extract remained neutral. From the organic phase the solvent was removeed *in vacuo* yielding 62 g (66 % of theory) oily reaction product D9.

Description 10

1-[(1-Ethoxycarbonyl)propyl]-4-[N-([1-ethoxycarbonyl] propyl)-N-methyl]amino piperidine (D10)

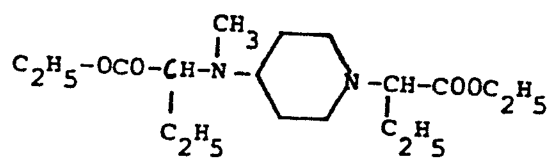

D10

To a suspension of 54 g (0.85 mol) $Na(CN)BH_3$ in 300 ml acetonitrile was added 37.5 g (1.24 mol) formaldehyde (95 ml 37% solution). This mixture was cooled with ice-bath and under stirring 53.4 g (0.17 mol) D9 diluted with 100 ml acetonitrile was slowly added dropwise (exothermic reaction). The reaction mixture was stirred for 2 days at room temperature until the reaction was complete. The solid part was filtered off and the solvent evaporated *in vacuo*. The residue was neutralized with saturated $NaHCO_3$ solution and extracted with diethylether (500 ml). In order to separate the impurities, the ether extract was washed with 6 x 100 ml $Na_2CO_3$ solution. The extract was then dried over $Na_2SO_4$ and the solvent evaporated yielding 50 g (90% of theory) D10.

Description 11

1-[2-(1-oxy-butyl)]-4-[N-methyl-N-(2-[1-oxy-butyl])] amino-piperidine (D11)

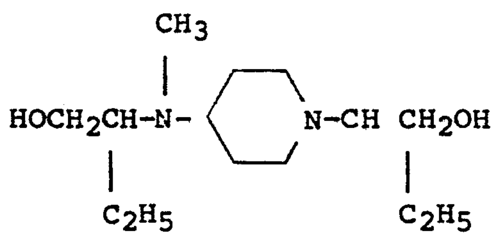

D11

45.92 g (0.14 mol) D10 was reduced with $LiAlH_4$ analogously to the process of description 4, yielding 28.6 g (79% of theory) D11 as a yellow oil.

Description 12

1-[2-(1-chloro-butyl)]-4-[N-methyl-N-(2-[1-chloro-butyl])]-amino-piperidine (D12)

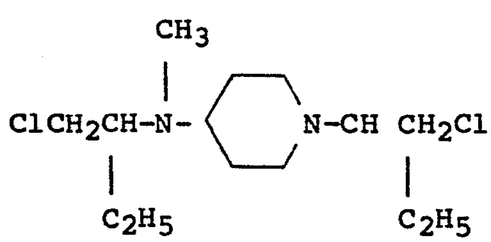

D12

To a cooled and stirred mixture of 44.9 g (0.38 mol) of thionylchloride and 50 ml toluene was slowly dropped a solution of 12.9 g (0.05 mol) D11 in 50 ml toluene. After the addition was complete, the reaction mixture was heated for 2 hours under reflux, cooled and poured onto 1000 g crushed ice and made basic with $NaHCO_3$ solution. The organic phase was separated and dried over $NaSO_4$. The solvent was evaporated, yielding 4.5 g (30% of theory) D12.

Description 13

1-[2-(1-amino-butyl)]-4-[N-methyl-N-(2-[1-amino-butyl])]amino-piperidine (D13)

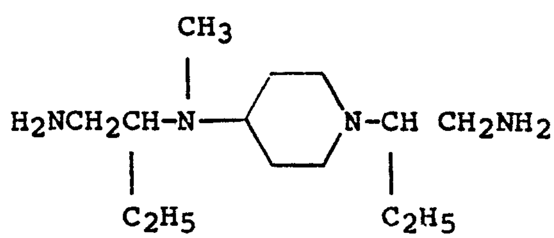

D13

A mixture of 4.5 g (0.015 mol) D12, 50 ml methanol and 50 ml 25% - ammonium hydroxide solution was stirred for 2 days at room temperature until the reaction was complete. The resulting solid was filtered off, the solvent evaporated and the residue dissolved in 50 ml water. This solution was made basic with dilute NaOH solution and extracted with 3 x 20 ml methylene chloride. The combined extracts were dried over $Na_2SO_4$ and evaporated to dryness, yielding 2.6 g (66% of theory) D13.

Description 14

N,N-dimethyl-N,N-di-[2-(1-amino)-pentyl]-ethylenediamine (D14)

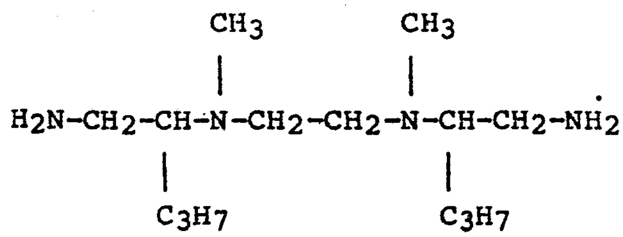

Compound D14 was prepared from N,N-dimethyl-N,N-ethylene-bis-(2-amino-pentane-nitrile) by procedures analogous to those of Descriptions 1 and 2.

Description 15

N,N-ethylene-bis-(2-hydroxymethyl-piperidine) (D15)

A mixture of 50g (0.404 mol) of 2-hydroxy-methyl-piperidine and 10 g (0.101 mol) 1,2-di-chloro-ethane was heated on an oil bath at 170°C for 1 hour. After cooling, the solidified reaction mixture was suspended in 150 ml toluene. Then 150 ml water was added, made basic with $Na_2CO_3$ solution and gently shaken in a separation funnel. The toluol phase was separated, washed twice with 100 ml water and evaporated to dryness. The residue (19 g) was stirred for 1 hour with 150 ml petroleum-ether. The resultant precipitate was separated by suction and recrystallized from petroleum-ether giving 6.8 g white crystals of less polar reaction product D15a. M.pt.: 108°C.

The combined water phases were evaporated to dryness and suspended in 100 ml petroleum-ether. The inorganic solid was filtered off. The filtrate was combined with the mother liquors of D15a. The solvent was removed in vacuo. The residue was dissolved in 50 ml methanol and acidified with methanolic HCl to pH 1, yielding 9.0 g of the hydrochloride of more polar reaction product D15b. M.pt.: 258°C (free base M.pt.: 62°C).

Both products are mixtures of diastereomers.

D15a: $C_{14}H_{28}N_2O_2$
mW: 256.39
M.pt.: 108°C

Elemental analysis:

| | C | H | N | O |
|---|---|---|---|---|
| calc. | 65.59 | 11.01 | 10.93 | 12.48 |
| found | 65.68 | 10.99 | 10.82 | 12.52 |

D15b: $C_{14}H_{28}N_2O_2 \cdot 2HCl$
MW: 329.31
M.pt.: 258°C

Elemental analysis:

| | C | H | N | O | Cl |
|---|---|---|---|---|---|
| calc. | 51.06 | 9.18 | 8.51 | 9.72 | 21.53 |
| found | 51.90 | 9.04 | 8.14 | 10.80 | 21.10 |

## Description 16

### N,N-ethylene-bis-(2-chloromethyl-piperidine) (D16)

To a cooled and stirred solution of 12 g (0.0 mol) D15a in 25 ml of chloroform, 41.2 g (0.346 mol) of thionyl chloride were added dropwise. The reaction mixture was heater for 20 min under reflux. The solvent and excess of thionyl chloride was evaporated in vacuo. The residue was hydrolyzed with 15 ml water and extracted with 50 ml toluene (to separate unreacted starting material). The residue was dissolved in 50 ml water, made alkaline with $Na_2CO_3$ solution to pH 8 and extracted with 100 ml petroleum-ether. The organic layer was separated and the solvent removed in vacuo giving 13.2 g (96% of theory) TLC-pure oily product D16a.

The more polar intermediate D15b reacted analogously with thionyl chloride yielding solid product D16b. M.pt.: 63° C. TLC, elemental analysis and NMR spectrum are identical with D16a.

$C_{14}H_{26}N_2Cl_2$

MW:

M.pt.: D16a (oil), D16b (63°C)

Elemental analysis:

| | C | H | N | Cl |
|---|---|---|---|---|
| calc. | 57.34 | 8.94 | 9.55 | 24.18 |
| D16a:found | 57.34 | 8.90 | 9.62 | 23.98 |
| D16b:found | 57.31 | 8.90 | 9.60 | 24.80 |

## Description 17

### N,N′-ethylene-bis-(2-amino-methyl-piperidine) (D17)

A mixture of 12 g (0.04 mol) of D16a, 27g 25% ic $NH_4OH$ (0.41 mol) and 300 ml methanol was stirred for 48 hours at room temperature. The solvent was removed in vacuo, and the residue dissolved in 20 ml water. The solution was made alkaline to pH 8 with $Na_2CO_3$ solution and extracted with chloroform. The chloroform phase was separated. The water phase was made alkaline to pH 12 and extracted with 3 x 50 ml chloroform. The combined chloroform extracts were dried over $Na_2SO_4$ and evaporated to dryness giving 8.4 g of oily (TLC-pure) product D17.

## Example 1

**N,N'-dimethyl-N,N'-di-[1-(3,4,5-trimethoxybenzamido)-butyl-(2)]-ethylenediamine (E1)**

### Method(a)

A solution of D2 (5 g, 0.025 mol) and triethylamine (5 2g, 0.025 mol) and triethylamine (5.2g, 0.055 mol) in dioxane (70 ml) was added dropwise to a solution of 3,4,5-trimethoxybenzoylchloride (12.6g, 0.055 mol) in dioxane (80 ml) at room temperature. Thereafter the mixture was heated on oil at 50°C and stirred for a further four hours until the reaction was complete. The solvent was evaporated and the crude product was dissolved in chloroform (200 ml). The solution was displaced in a separation funnel and washed twice with $Na_2CO_3$ solution (6g in 100 ml water). The separated organic layer was dried over $Na_2SO_4$ and solvent was evaporated. The residue was dissolved in hot methanol (200 ml). The product E1 was crystallized from the solution (3.9 g) (yield 25% of theory). M.pt. 176°C.

### Method(b)

To a solution of 30.7 g (0.13 mol) 3,4,5-trimethoxybenzoychloride in 300 ml dioxane, a solution of 15 g (0.065) D2 in 50 ml dioxane was added dropwise under stirring and heating to 40°C until the reaction was complete. The solvent was removed *in vacuo* and the residue was dissolved in 200 ml water. The solution was neutralised with $Na_2CO_3$ and extracted with 2 x 100 ml chloroform. After the solvent was removed the oily residue crystallized, yielding 60 g crude product E1 which was purified by repeated recrystallisation from methanol and ethylacetate. Yield 26.6 g (66.2% of theory). M.pt. 176°C.

Empirical formula $C_{32}H_{50}N_4O_8$ M.Wt. 618.77

| Elem.Anal. | C | H | N | O |
|---|---|---|---|---|
| calc: | 62.12 | 8.14 | 9.05 | 20.69 |
| found: | 62.00 | 8.2 | 8.84 | 21.03 |

Example 2

N,N'-dimethyl-N,N'-di-(2-[1-(3,5-dichlorobenzamido)]-butyl)-ethylene-diamine (E2)

To a cooled solution of 9.2 g (0.043 mol) 3,5-dichlorobenzoylchloride in 80 ml dioxane, a solution of 5 g (0.021 mol) D2 in 20 ml dioxane was added dropwise under stirring. The reaction was complete after 4 hours. The solvent was removed *in vacuo* and the residue was dissolved in 150 ml water, neutralized with $Na_2CO_3$ and extracted with 2 x 50 ml chloroform. After the solvent was removed, the crude product was purified by filtration through 50 g silica gel column, eluted with methylenechloride and a 98:2 mixture of methylenechloride: methanol. The eluate was evaporated and the residue crystallised from ethylacetate yielding 2.9 g (23% of theory) pure E2.

Empirical formula $C_{26}H_{34}N_4O_2Cl_4$ M.Wt. 576.4
M.pt. 160°C

| Elem.Anal. | C | H | N | O | Cl |
|---|---|---|---|---|---|
| calc.: | 54.18 | 5.95 | 9.72 | 5.55 | 24.6 |
| found: | 54.13 | 6.02 | 9.89 | 5.79 | 24.57 |

## Example 3

1-[1-(3,4,5-trimethoxybenzamido-butyl-(2))]-4-N-methyl-N-[1-(3,4,5-trimethoxybenzamido-butyl-(2))]-amino-piperidine (Compound E38)

To a cooled solution of 5.1 g (0.022 mol) 3,4,5-trimethoxybenzoyl chloride in 60 ml toluene and 2.23 g (0.022) mol) triethylamine was slowly dropped a solution of 2.6 g D13 in 20 ml toluene. The reaction mixture was stirred for 2 hours at room temperature. The formed precipitate was filtered off and the filtrate washed with 3 x 30 ml dilute NaOH solution. The toluene solution was then dried over $Na_2SO_4$ and evaporated to dryness. For purification the residue was dissolved in methylene chloride and chromatographed over silica gel column.

Yield: 0.5g E38

| Elem.Anal. | C | H | N | O |
|---|---|---|---|---|
| calc.: | 61.63 | 8.16 | 8.46 | 21.75 |
| found: | 62.18 | 7.83 | 8.03 | 21.96 |

## Example 4

N,N′-tetramethyl-N,N′-di-[1-(3,4,5-trimethoxybenzamido)-butyl-(2)]-ethylene-di-ammonium iodide (E15)

A mixture of 3.4 g (0.005 mol) of E 1, 6.0 g methyl iodide (0.042 mol) and 100 ml acetonitrile was heated under reflux for 4 hours. The solvent was evaporated in *vacuo*. The residue (3.1 g) was stirred with 50 ml chloroform. The resultant solid was separated by suction and recrystallized from 20 ml hot methanol yielding 1.2 g white crystals.

M.pt.: 214°C (decomp.)

MW: 902.65 $C_{34}H_{56}I_2N_4O_8$

Elemental analysis:

| | C | H | N | I | O |
|---|---|---|---|---|---|
| calc. | 45.24 | 6.25 | 6.21 | 28.12 | 14.18 |
| found | 45.39 | 6.29 | 6.04 | 28.28 | 14.01 |

Example 5

N,N′-ethylene-bis-[2-(3,4,5-trimethoxybenzamido)-piperidine] (E16)

To a solution of 9.2 g (0.041 mol) of 3,4,5-trimethoxybenzoyl-chloride in 50 ml dioxane, a solution of 4.5 g (0.0177 mol) of D17 in 50 ml dioxane was added dropwise under stirring and heating to 50°C. After 4 hours, the reaction was complete. The solvent was evaporated in vacuo and the residue dissolved in 100 ml water. The solid 3,4,5-trimethoxybenzoic acid was filtered off. The filtrate was extracted with chloroform to separate unreacted acid chloride. The water phase was made alkaline to pH 10 and extracted with 100 ml chloroform. The chloroform phase was evaporated to dryness and the residue, dissolved in 20 ml chloroform, was purified over 100 g silica gel column. The reaction product was eluted with chloroform and a mixture of chloroform/methanol (98:2). 1.2 g TLC-pure reaction product E15 was isolated. M.pt.: 213°C, $[\alpha]^{20}_{D}$= +1.95 (1% -chloroform/methanol 1:1)

$C_{34}H_{50}N_4O_8$

MW: 642.79

**Elemental analysis:**

| | C | H | N | O |
|---|---|---|---|---|
| calc. | 63.53 | 7.84 | 8.72 | 19.91 |
| found: | 63.32 | 7.72 | 8.95 | 20.20 |

**Example 6**

**N,N′-dimethyl-N,N′-di[1-nicotinoylamido-butyl-(2)]-ethylene-diamine (E30)**

To a solution of 5.35 g nicotinoylchloride hydrochloride (0.03 mol) in 30 ml toluene, a solution of 3.45 g D2 (0.015 mol) and 6.1 g (0.06 mol) triethylamine in 20 ml toluene was added dropwise at room temperature. The resultant solid was filtered off and the filtrate extracted with 100 ml water. The toluene phase was evaporated to dryness. The water phase was acidified with dilute HCl to pH 1 and extracted with toluene to remove impurities. Then the water phase was made alkaline with a dilute NaOH to pH 12 and extracted with methylene chloride to separate additional amounts of reaction product. The methylene chloride phase was dried over $Na_2SO_4$ and combined with the residue from the toluene mother liquor. The solvent was evaporated and the residue (4 g) was purified over 50 g silica gel column eluted with $CH_2Cl_2$: MeOH 10.:0.5) yielding 2.41 g (39% of theory) of E30 base. The base was dissolved in 117 ml of 0.2 N isopropanolic HCl solution yielding 2.4 g of E30 dihydrochloride.

| | |
|---|---|
| | $C_{24}H_{38}N_4O_2Cl_2$ |
| M.pt.: | 224 - 226$^{o}$C (decomp.) |
| MW: | 485 |

Example 7

N,N′-dimethyl-N,N′-di[1-(3,4,5-trimethoxycinnamoyl-amido)-butyl-(2)-ethylene-diamine (E35)

To a solution of 5.28 g (0.021 mol) of 4-nitro-1,8-naphthosultone in 40 ml dimethylformamide, a solution of 5.0 g (0.021 mol) of 3,4,5-trimethoxycinnamic acid and 2.13 g (0.021 mol) of triethylamine in 40 ml dimethylformamide was added dropwise under stirring at room temperature. After 3 hours, a solution of 2.4 g (0.0105 mol) of D2 in 10 ml dimethylformamide was added, and the reaction mixture was stirred for an additional 2 hours. The solvent was evaporated in vacuo. The residue was dissolved in 300 ml 7%ic $NaHCO_3$ solution and extracted twice with 300 ml ethyl acetate. The organic layer was separated, dried over $Na_2SO_4$ and evaporated to dryness. The residue was purified over 100 g silica gel column. The reaction product was eluted with a mixture of methylenechloride/ methanol (97:3) yielding 0.7 g TLC-pure product E35.

| | |
|---|---|
| | $C_{36}H_{54}N_4O_8$ |
| M.pt.: | 158°C |
| MW: | 670.85 |

Compounds E9 and E11 of Table IA were prepared from intermediates D7 and D2 respectively in a similar manner to the method of Example 1(a).

Compounds E3, E4, E5, E6, E7, E10, E11 E12 and E17 of Table IA were prepared from intermediate D2 in a similar manner to the method of Example 1(b).

Compound E13 and E14 of Table IA was prepared from intermediate D14, in a similar manner to the method of Example 1(b).

Compound E8 was prepared from N,N'-di-[2-(S)-(1-oxybutyl)]-ethylenediamine (prepared from (S)-2-aminobutanol in accordance with the procedures of British Patent 961317) by successive procedures analogous to those of Descriptions 4,5,6 and 7 and Example 2.

Compounds E18, E19, E20, E21, E22 and E28 of Table IB are prepared from intermediate D2 in a similar manner to the methods of Example 1 or 2.

Compounds E23, E24, E25 and E27 of Table IB are prepared from intermediate D7 in a similar manner to the methods of Example 1 or 2.

Compound E25 of Table IB is prepared analogously to compounds E13, E23, E24, E25 and E27.

Compound E29 of Table IIA was prepared from intermediate D2 in a similar manner to the method of Example 1(b).

Compounds E31 and E32 of Table IIB are prepared from intermediate D7 and compound E33 from intermediate D2 analogously to compound E29.

Compound E34 of Table IIIA and compounds E36 and E37 of Table IIIB are prepared from intermediate D2 in a similar manner to the method of Example 1(b).

Compounds E39 and E40 of Table IV are prepared analogously to compound E38 in a similar manner to the method of Example 3.

## Table IA

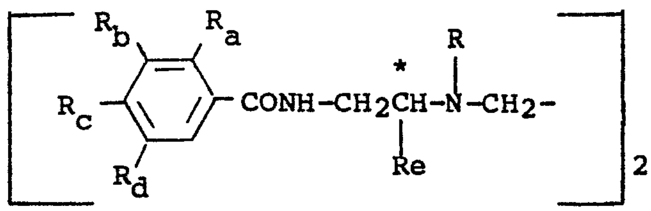

| Compound No. | Ra | Rb | Rc | Rd | Re | R | Salt | % Yield | Mp. °C |
|---|---|---|---|---|---|---|---|---|---|
| E1 method 1a<br>method 1b | H | $OCH_3$ | $OCH_3$ | $OCH_3$ | $C_2H_5$ | $CH_3$ | - | 25<br>66 | 176<br>176 |
| E2 | H | Cl | H | Cl | $C_2H_5$ | $CH_3$ | - | 23 | 160 |
| E3 | H | Cl | H | H | $C_2H_5$ | $CH_3$ | - | 22 | 138 |
| E4 (isomer a) | H | Cl | Cl | H | $C_2H_5$ | $CH_3$ | - | 17 | 116 |
| E5 (isomer b) | H | Cl | Cl | H | $C_2H_5$ | $CH_3$ | - | 9 | 124 |
| E6 | H | $CH_3$ | $CH_3$ | H | $C_2H_5$ | $CH_3$ | - | 36.5 | 130 |
| E7 | H | H | CN | H | $C_2H_5$ | $CH_3$ | HCl | 61 | 165 |
| E8, (S, $S^1$ enantiomer | H | $OCH_3$ | $OCH_3$ | $OCH_3$ | $C_2H_5$ | $CH_3$ | - | | 161-163 |
| E9 | H | $OCH_3$ | $OCH_3$ | $OCH_3$ | 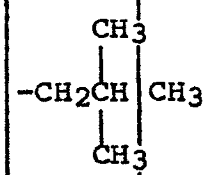 | $CH_3$ | - | 8 | 155 |
| E10 | H | $OCH_3$ | H | $OCH_3$ | $C_2H_5$ | $CH_3$ | - | 8 | 142 |
| E11 method 1a<br>method 1b | H | $OCH_3$ | $OCH_3$ | H | $C_2H_5$ | $CH_3$ | HCl | 10<br>36 | 134 IPA/EE<br>136 |
| E12 | H | $-O-CH_2-O-$ | | H | $C_2H_5$ | $CH_3$ | HCl | 34 | 145 |

TABLE IA continued

| Compound No. | Ra | Rb | Rc | Rd | Re | R | Salt | % Yield | Mp. °C |
|---|---|---|---|---|---|---|---|---|---|
| E13 (isomer a) | H | $OCH_3$ | $OCH_3$ | $OCH_3$ | $C_3H_7$ | $CH_3$ | | 14 | |
| E14 (isomer b) | H | $OCH_3$ | $OCH_3$ | $OCH_3$ | $C_3H_7$ | $CH_3$ | | 5 | |
| E15 | H | $OCH_3$ | $OCH_3$ | $OCH_3$ | $C_2H_5$ | $CH_3$ | $CH_3I$ | 26.6 | 214 |
| E16 | H | $OCH_3$ | $OCH_3$ | $OCH_3$ | $-(CH_2)_4-$ | | - | 11 | 213 |
| E17 | H | H | $OCH_3$ | H | $C_2H_5$ | $CH_3$ | HCl | 49 | 151 |

Table IB

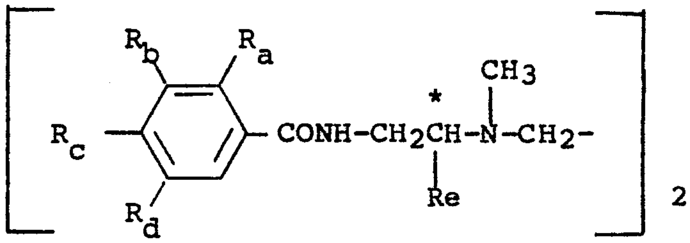

| Compound No. | Ra | Rb | Rc | Rd | Re |
|---|---|---|---|---|---|
| E18 | H | $CH_3$ | H | $CH_3$ | $C_2H_5$ |
| E19 | H | H | F | H | $C_2H_5$ |
| E20 | $CH_3$ | H | H | H | $C_2H_5$ |
| E21 | $CH_3$ | $CH_3$ | H | H | $C_2H_5$ |
| E22 | H | $CH_3$ | H | H | $C_2H_5$ |
| E23 | H | $OCH_3$ | H | $OCH_3$ | $CH_2-CH(CH_3)_2$ |
| E24 | H | Cl | H | H | $CH_2-CH(CH_3)_2$ |
| E25 | H | $-O-CH_2-O-$ | | H | $CH_2-CH(CH_3)_2$ |
| E26 | H | Cl | H | H | $C_3H_7$ |
| E27 | H | H | F | H | $CH_2-CH(CH_3)_2$ |
| E28 | H | $OCH_3$ | H | H | $C_2H_5$ |

Table IIA

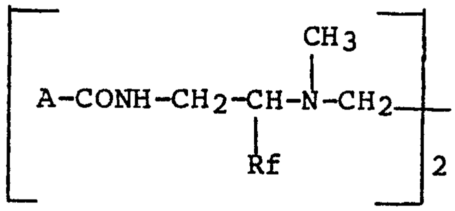

| Compound No. | A | Rf | Salt | Yield | Mp°C |
|---|---|---|---|---|---|
| E29 | 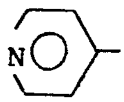 | $C_2H_5$ | - | 24 | Oil |
| E30 | 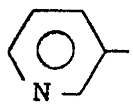 | $C_2H_5$ | HCl | 39 | 224-226 |

Table IIB

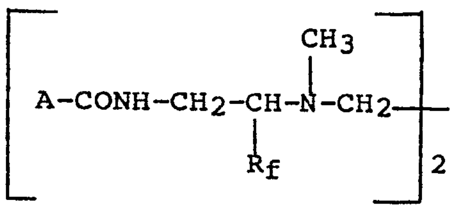

| Compound No. | A | Rf |
|---|---|---|
| E31 | 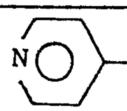 | 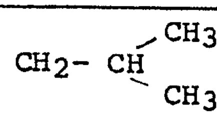 |
| E32 | 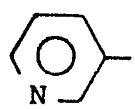 | 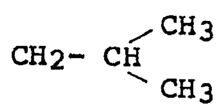 |
| E33 | 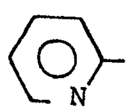 | $C_2H_5$ |

Table IIIA

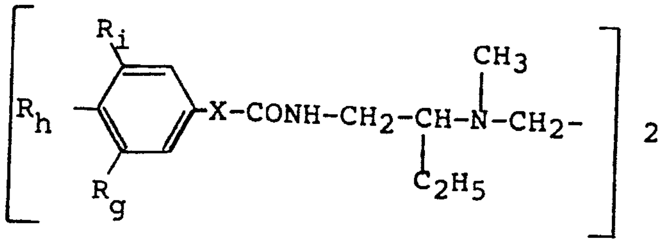

| Compound No. | Rg | Rh | Ri | X | Salt | Yield | Mp.°C |
|---|---|---|---|---|---|---|---|
| E 34 | $OCH_3$ | $OCH_3$ | $OCH_3$ | $-CH_2-$ | - | 32.5 | 142 |
| E 35 | $OCH_3$ | $OCH_3$ | $OCH_3$ | -CH=CH- | - | 24 | 158 |

Table IIIB

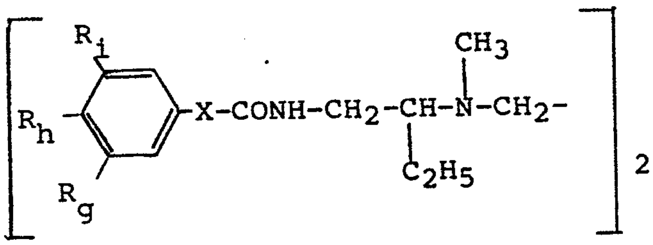

| Compound No. | Rg | Rh | Ri | X |
|---|---|---|---|---|
| E 36 | Cl | H | H | $-CH_2-$ |
| E 37 | $OCH_3$ | $OCH_3$ | H | $-CH_2-$ |

Table IV

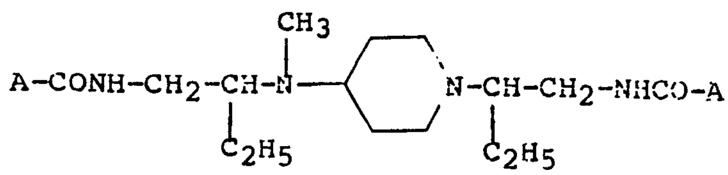

| Compound No. | A | Salt | Yield | Mp$^{o}$C |
|---|---|---|---|---|
| E38 | 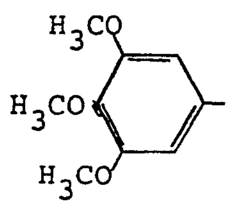 | HCl | 53 | 205-208 |
| E 39 | 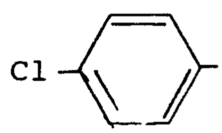 | | | |
| E 40 | 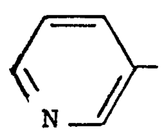 | | | |

SPECTRAL DATA

NMR - (DMSO)

E1 $C_{32}H_{50}N_4O_8.2Hcl$
0.8 - 1.2 (m,6H); 1.3 - 2.2 (m,4H); 2.84 (s,6H) 3.2 - 4.05 (m + 2s(3.83;3.67), 28H); 7.32 (s,4H) 9.1 (br., 2H) exch with $D_2O$; 10.5(br.,2H) exch with $D_2O$

E11 $C_{30}H_{46}N_4O_6.2HCl$
1.03(m,6H); 1.25 - 2.2(m,4H); 2.35(s, 6H) 3.15 - 4.4(m+2s(3.80;3.82),22H);6.85 - 7.1 (m,2H) 7.5 - 7.8(m,4H); 9.0(br.;2H) exch with $D_2O$; 10.8 (br.,2H) exch with $D_2O$

E12 $C_{28}H_{38}N_4O_6.2Hcl$
1.03 (t,j:6Hz;6H);1.3 - 2.2(m,4H);2.84(s,6H)3.1 - 4.1(m,10H);6.09(s,4H);6.9 - 7.7(m,6H) 8.9(br.,2H) exch with $D_2O$;10.8 (br.,2H) exch with $D_2O$

E14 $C_{34}H_{56}N_4O_8I_2$
1.13(t,j:6Hz,6H);1.35 - 2.25(m,4H) 2.9 - 4.3 (m + 3s(3.27;3.72;3.83),40H) 7.2(s,4H); 8.8(br.,2H) exch with $D_2O$

E16 $C_{28}H_{42}N_4O_4.2HCl$
1.04(m,6H);1.3 - 2.25(m,4H);2.85(s,6H)3.05 - 4.2(m + s(3.81),16H);7.48(ABq,j:9Hz,8H)8.92(br.,2H) exch with $D_2O$; 10.8(br.,2H) exch with $D_2O$

<u>NMR ($CDCl_3$)</u>

E2 $C_{26}H_{34}N_4O_2Cl_4$
0.95(t,j:7Hz,6H);1.15 - 1.9(m,4H) 2.1 - 3.2(m + s(2.3),14H);3.6 - 4.0 (m,2H)7.1(br.,2H) exch with $D_2O$;7.3 - 7,6(m,6H)

E3 $C_{26}H_{36}Cl_2N_4O_2$
0.95(t,j:6Hz,6H);1.2 - 2.05(m,4H)2.1 - 3.15 (m + s(2.28),14H);3.85 - 4.25(m,2H)7.15 - 7.7(m,10H);2H exch with $D_2O$

E4 $C_{26}H_{34}Cl_4N_4O_2$
0.96(m,6H);1.15 - 2.1(m,4H)2.15 - 3.2(m + s(2.29),14H);3.85 - 4.3(m,2H)7.2 - 7.8(m,8H),2H exch with $D_2O$

E5 $C_{30}H_{46}N_4O_2$
0.93(t,j:6H4H$_2$,6H);1.1 - 1.95(m,4H)2.0 - 3.2(m + 2s(2.24),26H)3.6 - 4.2(m,2H);6.9 - 7.65(m,8H)2H exch with $D_2O$

E6 $C_{26}H_{34}Cl_4N_4O_2$
0.94(t,j:7Hz,6H);1.15 - 2.0(m,4H)2.15 - 3.2(m + s(2.29),14H);3.55 - 4.0(m,2H)7.05(br.,2H) exch with $D_2O$;7.3 - 7.85(m,6H)

E9 $C_{36}H_{38}N_4O_8$
0.86 (d,j:6Hz,6H);0.92(d,j:6Hz,6H)1.0 - 2.0(m,6H);2.1 - 3.2(m + s(2.14),14H)3.65 - 4.1(m + s(3.86),20H);7.06(s,4H)7.45(br.,2H) exch with $D_2O$

E10 $C_{32}H_{46}N_4O_6$
0.94(t,j:6.3Hz,6H);1.1 - 1.95(m,4H)2.1 - 3.15(m + s(2.25),14H);6.4 - 6.55(m,2H)6.7 - 6.8(m,4H);7.2/br.,2H) exch with $D_2O$

E29 $C_{24}H_{36}N_6O_2$
0.94(t,j:6.5Hz,6H);1.15 - 2.0(m,4H)2.1 - 3.25(m + s(2.27),14H);3.6 - 4.1(m,2H)7.25(br.,2H) exch with $D_2O$;7.45 - 7.65(m,4H)8.6 - 8.8(m,4H)

E34 $C_{34}H_{54}N_4O_8$
0.88(t,j:6Hz,6H);1.05 - 1.8(m,4H)1.9 - 2.55(m + s,14H);2.6 - 3.0(m,2H)3.47(s,4H);3.81(s,6H);3.83(s,12H)6.48(s,4 H);6.4 - 6.8(2d,j:7Hz,2H) exch with $D_2O$

D2 $C_{12}H_{30}N_4$
0.92(t,j:6,4Hz,6H);1.0 - 1.9(m + s(1.44),8H),4H exch with $D_2O$2.1 - 2.8(2.24),16H)

D16a $C_{14}H_{26}N_2Cl$
1.13 - 1.92(m,12H);2,1-3,1(m,10H)3.4(br,4H)

D16b
1.08 - 2.03(m,12H);2.15 - 3.1(m,10H)3.4 - 3.85(m.4H)

D17 $C_{14}H_{30}N_4$
1.2 - 2.0(m,12H);2.0 - 3.4(m,14H)5.11(s,4H) exch with $D_2O$

E16 $C_{34}H_{50}N_4O_8$(diastereoisomers mixtures)
1.3 - 2.0(m,12H);2.0 - 3.1(m,12H)3.9(s,18H);4.0 - 4.4(m,2H)6.95(br,2H) exch with $D_2O$;7.0(s,4H)

1.2 - 2.0(m,12H);2.1 - 3.2(m,10H)3.35 - 3.65(m,2H;3,89(s,18H)4.05 - 4.4(m,2H);6.6 - 7.0(br.,2H) exch with $D_2O$;7.03(s,4H)

## PHARMACOLOGICAL DATA

### 1. Antiarrhythmic activity

Compound (E1) infused at 2.5mg/min in the ear vein of conscious rabbits inhibited the arrhythmogenic effect of suprathreshold cardiac electric stimulation. The therapeutic index, i.e. the ratio between the dose of compound required to produce A-V-block (8 mg/kg) and that dose which gave an antiarrhythmic effect (0.9mg/kg) was 8.9.

### 2. Antianginal activity

Compound (E1) reduced ST-segment elevation in the ECG of anaesthetized rats following i.v-injection of 1 I.U./kg vasopressin significantly, the minimum effective i.v. antianginal dose being 0.2 mg/kg.

### 3. $Ca^{++}$-antagonistic activity

In guinea-pig papillary muscle (34$\pm$ 1$^{o}$C) *in vitro*, the Compound (E1) (1-3 x $10^{-6}$M) depressed the maximal rate of rise ($V_{max}$) of calcium dependent action potentials evoked in krebs medium containing 26 nM $K^{+}$ and 0.5 nM, $BaCl_2$. These action potentials were recorded using conventional intracellular techniques.

### 4. Vasopressin test in anaesthetized rats

Vasopressin (Lysopressin: 1 I.U./kg) was administered intravenously in order to provoke coronary spasm. Compound (E1) was active against coronary spasm at a dose of 32mg/kg i.d. with a duration of action greater

than 2 hours. The ability of test compounds to reduce coronary spasm is taken as a measure of their anti-anginal potential in spasm - induced angina pectoris. (Ref. Drug.Res. 33(11), 8,1117-1121 (1983)).

5. Haemodynamic profile in anaesthetized rats

The effect of compounds of the invention on heart rate, systolic left ventricular pressure, left ventricular dp/dt max and mean arterial blood pressure was investigated so as to obtain information on the anti-anginal potential of these compounds, by reducing the oxygen demand of the ischaemic heart. Results are shown in Table V.

Table V

0174085

| Compound Number | haemodynamic effects (rat) | | | | i.v. duration of action on heart rate (rat) (min) |
|---|---|---|---|---|---|
| | $D_{hr}20$* | %effect of $D_{hr}20$ on | | | |
| | | LVP** | BP*** | $dp/dt_{max}$ | |
| E1 | 3.2 | -17 | | -24 | 5 |
| E2 | 3.2 | -5/30 | -7 | -21 | 15 |
| E3 | 1.6 | -2 | -16 | -15 | 10 |
| E5 | 1.6 | -6 | -2 | -15 | 10 - 15 |
| E7 | 3.2 | -5 | -6 | -15 | 5 - 10 |
| E8 | 3.2 | -29 | -30 | -36 | |
| E9 | 1.6 | -15 | -17 | -18 | 15 |
| E10 | 1.6 | -10 | | -16 | 5 - 10 |
| E11 | 3.2 | -4 | -2 | -7 | 5 - 10 |
| E12 | 1.6 | -8 | -4 | -11 | 10 - 15 |
| E16 | 6.4 | -17.3 | -17 | -32 | 10 |
| E29 | 12.8 | -6 | -10 | -2 | 10 |
| E30 | 12.8 | -9 | -14 | -15 | 10 |
| E38 | 12.8 | -7 | -6 | -32 | 10 - 15 |

*$D_{hr}20$ = i.v. dose which reduces heart rate by ca. 20%

**LVP = systolic left ventricular pressure

***BP = mean arterial blood pressure

Results

The above results demonstrate that the compounds of the invention have therapeutic potential in both stable and variant angina pectoris. Of special interest is the bradycardic activity of some test compounds.

The activity of compound E1 after intraduodenal administation suggests that the claimed compounds are well absorbed and/or show high metabolic stability.

Toxicity

No toxic effects were observed in the above tests.

Claims

1. A compound of formula (I) or a pharmaceutically acceptable salt thereof:

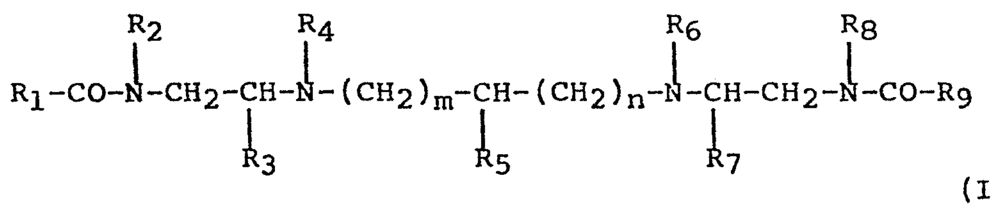

(I)

wherein:

$R_1$ and $R_9$ are independently phenyl, phenyl $C_{1-4}$ alkyl, phenyl $C_{2-4}$ alkenyl or pyridyl, the aromatic moiety optionally substituted by one, two or three of halogen, trifluoromethyl, $C_{1-4}$ alkoxy, $C_{1-4}$ alkyl, cyano, hydroxy, nitro, $NR_{10}R_{11}$ or $O_2SNR_{10}R_{11}$ wherein $R_{10}$ and $R_{11}$ are independently hydrogen or $C_{1-6}$ alkyl or together are $C_{3-6}$ polymethylene, or disubstituted at adjacent carbon atoms by $C_{1-2}$ alkylenedioxy; $C_{3-8}$ cycloalkyl; or aliphatic heterocyclyl having up to six ring atoms the heteroatom(s) being selected from oxygen, sulphur or nitrogen;

m and n are independently integers of 0 to 3, such that m + n is 0 to 3; and

$R_2$ and $R_8$ are independently hydrogen $C_{1-4}$ alkyl or phenyl $C_{1-4}$ alkyl, $R_3$ is $C_{1-4}$ alkyl optionally substituted by hydroxy or thiol or phenyl $C_{1-4}$ alkyl,

or together with $R_4$ is $C_{3-6}$ polymethylene, $R_4$ is hydrogen, $C_{1-4}$ alkyl, phenyl $C_{1-4}$ alkyl or as defined with $R_3$, or together with $R_5$ is $-(CH_2)_p-$ where p is an integer from 2 to 6 such that m + p is 3 to 6, $R_5$ is hydrogen, $C_{1-4}$ alkyl or as defined with $R_4$, or together with $R_6$ is $-(CH_2)_q-$ where q is an integer from 2 to 6 such that n + q is 3 to 6, $R_6$ is hydrogen, $C_{1-4}$ alkyl, phenyl $C_{1-4}$ alkyl or as defined with $R_5$, or together with $R_7$ is $C_{3-6}$ polymethylene, and $R_7$ is $C_{1-4}$ alkyl optionally substituted by hydroxy or thiol, phenyl $C_{1-4}$ alkyl or as defined with $R_6$, in which any phenyl moiety in $R_2$,$R_3$,$R_4$,$R_6$,$R_7$ and $R_8$ is optionally substituted by one or two of halogen, trifluoromethyl, $C_{1-4}$ alkoxy or $C_{1-4}$ alkyl.

2. A compound according to claim 1, wherein $R_1$ and $R_9$ are the same group.

3. A compound according to claim 1 or 2, wherein $R_2$ and $R_8$ are each hydrogen.

4. A compound according to any preceding claim, wherein $R_3$ and $R_7$ are the same and are ethyl, n-propyl or iso-butyl.

5. A compound according to any preceding claim of formula (II):

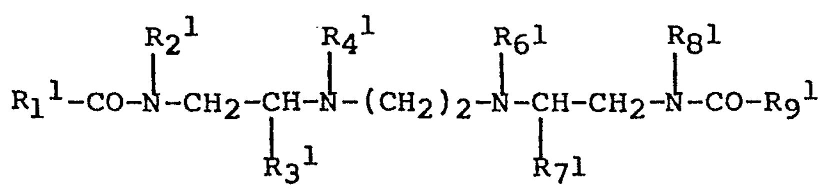

(II)

wherein:

$R_1{}^1$ and $R_9{}^1$ are pyridyl, phenyl, benzyl or styryl, optionally substituted in the phenyl ring as defined in claim 1;

$R_2{}^1$, $R_4{}^1$, $R_6{}^1$ and $R_8{}^1$ are hydrogen or $C_{1-4}$ alkyl; and $R_3{}^1$ and $R_7{}^1$ are $C_{1-4}$ alkyl.

6. A compound according to claim 5, wherein $R_4{}^1$ and $R_6{}^1$ are each methyl.

7. A compound according to any of claims 1 to 4, of formula (IIa)

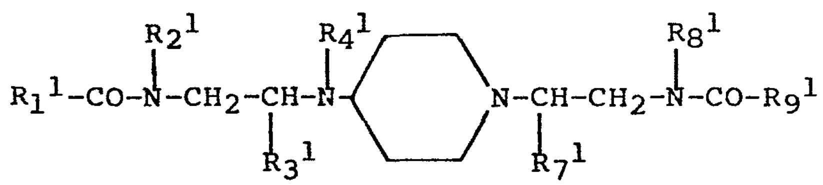

(IIa)

wherein $R_1{}^1$ to $R_4{}^1$ and $R_7{}^1$ to $R_9{}^1$ are as defined in claim 5.

8. A compound according to claim 7, wherein $R_4{}^1$ is methyl.

9. A compound according to claim 1 as described in Table IA, IB, IIA, IIB, IIIA, IIIB or IV.

10. A process for the preparation of a compound of formula (I) as defined in claim 1 or a pharmaceutically acceptable salt thereof, which process comprises reacting a compound of formula (III):

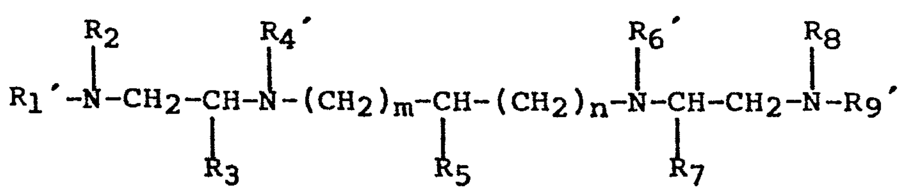

(III)

with a compound of formula (IV):

$$L\text{-}CO\text{-}R_{13} \qquad (IV)$$

wherein $R_4'$ and $R_6'$ are $R_4$ and $R_6$ or N-protecting groups, one of $R_1'$ and $R_9'$ is hydrogen and the other is $-COR_1$ or $-COR_9$ respectively or when $R_1$ and $R_9$ are the same group, $R_1'$ and $R_9'$ may both be hydrogen; $R_{13}$ is $R_9$ when $R_1$ is hydrogen and/or $R_1$ when $R_9$ is hydrogen and L is a leaving group, and thereafter removing $R_4'$ and/or $R_6'$ when protecting groups and/or optionally converting $R_2, R_4, R_6$ or $R_8$ when hydrogen to other $R_2, R_4, R_6$, or $R_8$ and/or optionally forming a pharmaceutically acceptable salt thereof.

11. A compound of formula (III):

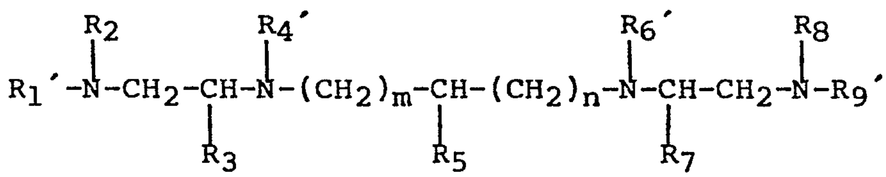

(III)

wherein $R_4'$ and $R_6'$ are $R_4$ and $R_6$ as defined in claim 1 or N-protecting groups, one of $R_1'$ and $R_9'$ is hydrogen and the other is $-COR_1$ or $-COR_9$ respectively where $R_1$ and $R_9$ are as defined in claim 1, or $R_1'$ and $R_9'$ may both be hydrogen, and the remaining variables are as defined in claim 1; with the proviso that when $R_1'$, $R_2$, $R_4'$, $R_5$, $R_6'$, $R_8$ and $R_9'$ are each hydrogen, $R_3$ and $R_7$ are each ethyl and m is 1, n is other than 0.

12. N,N'-dimethyl-N,N'-di-[2-(1-amino)-butyl]-ethylene-diamine; N,N'-dimethyl-N,N'-di-[2-(1-amino-4-methyl)pentyl]-ethylene-diamine, N,N′-dimethyl-N,N′-di-[2-(1-amino-pentyl]-ethylene-diamine; N,N′-ethylene-bis-(2-amino-methyl-piperidine); and 1-[2-(1-amino-butyl)]-4-[N-methyl-N-(2-[1-amino-butyl]))]-amino-piperidine.

13. A compound of formula (I) as defined in claim 1, or a pharmaceutically acceptable salt thereof, or a solvate of any of the foregoing, for use as an active therapeutic substance.

14. A compound of formula (I) as defined in claim 1, or a pharmaceutically acceptable salt thereof, or a solvate of any of the foregoing, for the treatment or prophylaxis of angina in mammals.

Claims

1. A process for preparing a compound of formula (I) or a pharmaceutically acceptable salt thereof:

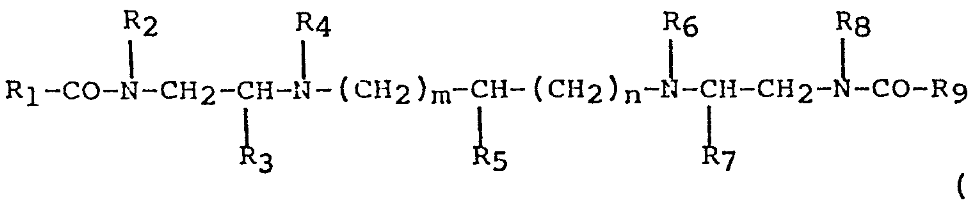

(I)

wherein:

$R_1$ and $R_9$ are independently phenyl, phenyl $C_{1-4}$ alkyl, phenyl $C_{2-4}$ alkenyl or pyridyl, the aromatic moiety optionally substituted by one, two or three of halogen, trifluoromethyl, $C_{1-4}$ alkoxy, $C_{1-4}$ alkyl, cyano, hydroxy, nitro, $NR_{10}R_{11}$ or $O_2SNR_{10}R_{11}$ wherein $R_{10}$and $R_{11}$ are independently hydrogen or $C_{1-6}$ alkyl or together are $C_{3-6}$ polymethylene, or disubstituted at adjacent carbon atoms by $C_{1-2}$ alkylenedioxy; $C_{3-8}$ cycloalkyl; or aliphatic heterocyclyl having up to six ring atoms the heteroatom(s) being selected from oxygen, sulphur or nitrogen;

m and n are independently integers of 0 to 3, such that m + n is 0 to 3; and

$R_2$ and $R_8$ are independently hydrogen $C_{1-4}$ alkyl or phenyl $C_{1-4}$ alkyl, $R_3$ is $C_{1-4}$ alkyl optionally substituted by hydroxy or thiol or phenyl $C_{1-4}$ alkyl, or together with $R_4$ is $C_{3-6}$ polymethylene, $R_4$ is hydrogen, $C_{1-4}$ alkyl, phenyl $C_{1-4}$ alkyl or as defined with $R_3$, or together with $R_5$ is $-(CH_2)_p-$ where p is an integer from 2 to 6 such that m + p is 3 to 6, $R_5$ is hydrogen, $C_{1-4}$ alkyl or as defined with $R_4$, or together with $R_6$ is $-(CH_2)_q-$ where q is an integer from 2 to 6 such that n + q is 3 to 6, $R_6$ is hydrogen, $C_{1-4}$ alkyl, phenyl $C_{1-4}$ alkyl or as defined with $R_5$, or together with $R_7$ is $C_{3-6}$ polymethylene, and $R_7$ is $C_{1-4}$ alkyl optionally substituted by hydroxy or thiol, phenyl $C_{1-4}$ alkyl or as defined with $R_6$, in which any phenyl moiety in $R_2$,$R_3$,$R_4$,$R_6$,$R_7$ and $R_8$ is optionally substituted by one or two of halogen, trifluoromethyl, $C_{1-4}$ alkoxy or $C_{1-4}$ alkyl;

which process comprises reacting a compound of formula (III):

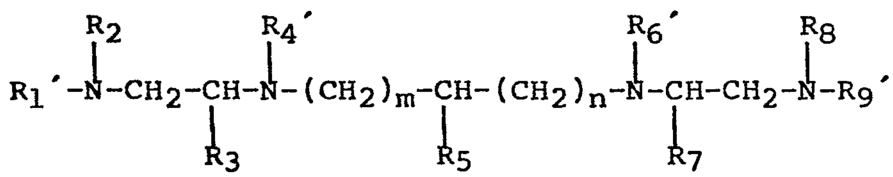

(III)

with a compound of formula (IV):

$$L-CO-R_{13} \qquad (IV)$$

wherein $R_4'$ and $R_6'$ are $R_4$ and $R_6$ or N-protecting groups, one of $R_1'$ and $R_9'$ is hydrogen and the other is $-COR_1$ or $-COR_9$ respectively or when $R_1$ and $R_9$ are the same group, $R_1'$ and $R_9'$ may both be hydrogen; $R_{13}$ is $R_9$ when $R_1$ is hydrogen and/or $R_1$ when $R_9$ is hydrogen and L is a leaving group, and thereafter removing $R_4'$ and/or

$R_6'$ when protecting groups and/or optionally converting $R_2, R_4, R_6$ or $R_8$ when hydrogen to other $R_2, R_4, R_6$, or $R_8$ and/or optionally forming a pharmaceutically acceptable salt thereof.

2. A process according to claim 1, wherein $R_1$ and $R_9$ are the same group.

3. A process according to claim 1 or 2, wherein $R_2$ and $R_8$ are each hydrogen.

4. A process according to any preceding claim, wherein $R_3$ and $R_7$ are the same and are ethyl, n-propyl or iso-butyl.

5. A process according to any preceding claim for preparing a compound of formula (II):

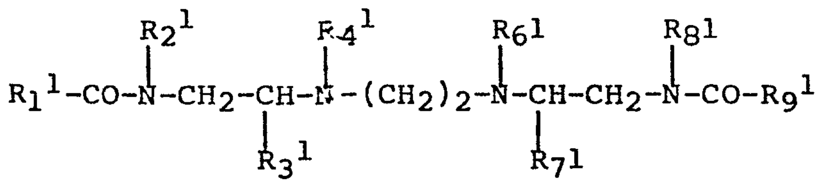

(II)

wherein:

$R_1{}^1$ and $R_9{}^1$ are pyridyl, phenyl, benzyl or styryl, optionally substituted in the phenyl ring as defined in claim 1;

$R_2{}^1$, $R_4{}^1$, $R_6{}^1$ and $R_8{}^1$ are hydrogen or $C_{1-4}$ alkyl; and $R_3{}^1$ and $R_7{}^1$ are $C_{1-4}$ alkyl.

6. A process according to claim 5, wherein $R_4{}^1$ and $R_6{}^1$ are each methyl.

7. A process according to any of claims 1 to 4, for preparing a compound of formula (IIa)

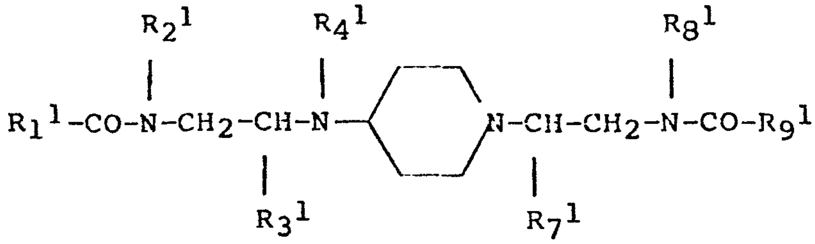

(IIa)

wherein $R_1{}^1$ to $R_4{}^1$ and $R_7{}^1$ to $R_9{}^1$ are as defined in claim 5.

8. A process according to claim 7, wherein $R_4{}^1$ is methyl.

9. A process according to claim 1 for the preparation of a compound as described in Table IA, IB, IIA, IIB, IIIA, IIIB or IV.

10. A process as claimed in claim 1 wherein the compound (III) is N,N′-dimethyl-N,N′-di-[2-(1-amino)-butyl]-ethylene-diamine; N,N′-dimethyl-N,N′-di-[2-(1-amino)-pentyl]-ethylene-diamine; N,N′-dimethyl-N,N′-di-[2-(1-amino-methyl)pentyl-ethylene diamine;N,N′-ethylene-bis-(2-amino-methyl-piperidine); or 1-[2-(1-amino-butyl)]-4-[N-methyl-N-(2-[1-amino-butyl])]-amino-piperidine.